(19) Europäisches Patentamt
European Patent Office
Office européen des brevets

(11) EP 3 927 308 B1

(12) **EUROPEAN PATENT SPECIFICATION**

(45) Date of publication and mention of the grant of the patent:
**21.09.2022 Bulletin 2022/38**

(21) Application number: **20704022.1**

(22) Date of filing: **10.02.2020**

(51) International Patent Classification (IPC):
***A61K 8/368*** (2006.01) ***A61K 8/40*** (2006.01)
***A61K 8/362*** (2006.01) ***A61Q 11/00*** (2006.01)
***A61Q 5/02*** (2006.01) ***A61Q 5/12*** (2006.01)
***A61Q 1/02*** (2006.01) ***A61Q 19/00*** (2006.01)
***A61Q 15/00*** (2006.01) ***A61Q 1/04*** (2006.01)
***A61Q 1/10*** (2006.01) ***A61Q 17/04*** (2006.01)
***A61Q 19/10*** (2006.01)

(52) Cooperative Patent Classification (CPC):
**A61K 8/368; A61K 8/362; A61K 8/40; A61Q 1/02; A61Q 1/04; A61Q 1/10; A61Q 5/02; A61Q 5/12; A61Q 11/00; A61Q 15/00; A61Q 17/04; A61Q 19/00; A61Q 19/10;** A61K 2800/524

(86) International application number:
**PCT/EP2020/053226**

(87) International publication number:
**WO 2020/169372 (27.08.2020 Gazette 2020/35)**

(54) **PERSONAL CLEANSING COMPOSITIONS COMPRISING A PRESERVATIVE SYSTEM**
PERSONENREINIGUNGSMITTEL ENTHALTEND EIN KONSERVIERUNGSYSTEM
COMPOSITIONS DE NETTOYAGE PERSONNEL COMPRENANT UN SYSTEME PRESERVATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.02.2019 EP 19157769**

(43) Date of publication of application:
**29.12.2021 Bulletin 2021/52**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **UNILEVER GLOBAL IP LIMITED**
  **Wirral**
  **Merseyside CH62 4ZD (GB)**
  Designated Contracting States:
  **CY DE GB IE IT MT RS TR**

(72) Inventors:
- **KADAMKODE, Vinitha**
  **Bangalore 560 066 (IN)**
- **MITRA, Rupak**
  **Bangalore 560 066 (IN)**
- **POINTON, Thomas, Richard**
  **Wirral Merseyside CH63 3JW (GB)**
- **STOTT, Ian, Peter**
  **Wirral Merseyside CH63 3JW (GB)**

(74) Representative: **Fijnvandraat, Arnoldus**
**Unilever N.V.**
**Unilever Patent Group**
**Bronland 14**
**6708 WH Wageningen (NL)**

(56) References cited:
**CN-A- 107 935 092**
**CN-B- 104 278 528**
**US-A- 3 446 630**
**US-A- 5 250 299**
**US-A1- 2018 177 695**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **AMMENDOLA S ET AL: "Acyl hydroxamate-based biocides and their use in disinfection and preservation systems", CA,, 18 December 2007 (2007-12-18), XP002719445,**

## Description

### Field of the invention

The present invention relates to a personal cleansing composition comprising a preservative system as defined in the appended claims and a surfactant.

### Background of the invention

The consumer goods industry has a constant need for agents having antimicrobial properties, in particular for the preservation of products which are otherwise perishable (such as e.g. cosmetics, pharmaceutical products or foodstuffs). It is preferred that such preservation chemicals that are from natural sources, that are abundant and readily available.

A large number of antimicrobial active compounds are already employed in the personal care industry, but alternatives nevertheless continue to be sought. Not all antimicrobial agents have preservative properties and thus the need for new preservation chemicals is particularly required. It is to be noted that the substances used in the personal care field must be toxicologically acceptable, readily tolerated by the skin, stable in the compositions being incorporated in, are colourless and odourless, inexpensive to prepare, have low ecological significance and easy to formulate.

The present application has found a preservative system suitable for use with consumer goods, particularly personal cleansing compositions. The preservative system is cost effective and easy to formulate with compositions of the invention. It comprises a combination of a well known and widely used preservative sodium benzoate in combination with certain aliphatic or aromatic short chain compounds have an acid group e.g. benzhydroxamic acid or tricarballylic acid (see for example US-A-5250299 for tricarballylic acid, US- A-3446630 for benzohydroxamic acid and US-A-2018177695 for sodium benzoate).

Further, it is expected that many conventional preservatives like parabens, methyl isothiazolinone (MIT), methyl chloro isothiazolinone (CIT) and hydantoins may face restrictions on their use by regulatory authorities in various countries in the years to come. Therefore there has been a recent spate of research in identifying new preservative actives or combination of actives that provide enhanced preservative activity in inhibiting growth of microorganisms in pharmaceutical/ cosmetic compositions.

It is thus an objective of the present invention to provide for an effective preservative system comprising two actives which interact synergistically to deliver enhanced activity at low concentrations.

### Summary of the invention

The present invention relates to a composition comprising

(a) a preservative system comprising sodium benzoate in combination with a second active selected from benzhydroxamic acid or tricarballylic acid;and
(b) a surfactant;

wherein the weight ratio of the second active to sodium benzoate is in the range of 1:8 to 1:2 when the second active is benzhydroxamic acid;
and wherein the weight ratio of the second active to sodium benzoate is in the range of 1:8 to 4:1 when the second active is tricarbalyllic acid;
wherein the composition is a personal cleansing composition.

According to another aspect of the present invention there is provided a method of preserving a composition as defined above comprising the step of adding to the composition a preservative system of the invention.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed

in the format "from x to y" are understood to include x and y. When for a specific feature multiple preferred ranges are described in the format "from x to y", it is understood that all ranges combining the different endpoints are also contemplated.

The preservative system comprises sodium benzoate in combination with a second active selected from benzhydroxamic acid or tricarballylic acid. These compounds are described in more detail below.

Sodium benzoate having the following structure has been known and used as a preservative to inhibit growth of microorganisms in compositions containing it.

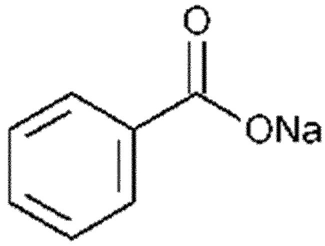

Sodium Benzoate

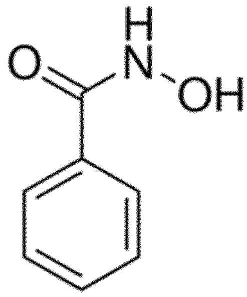

benzhydroxamic acid

Tricarballylic acid is also known as β carboxy glutaric acid and has the structure as given below:

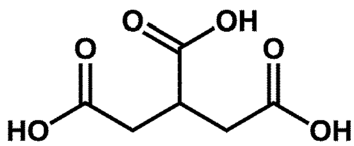

tricarballylic acid (β carboxy glutaric acid)

In the preservative system, the weight ratio of benzhydroxamic acid and sodium benzoate is in the range of 1:8 to 1:2 when the second active is benzhydroxamic acid. The weight ratio of tricarballylic acid to sodium benzoate is in the range of 1:8 to 4:1 when the second active is tricarbalyllic acid.

The present invention provides for a composition comprising the preservative system of the invention. The composition of the invention is a personal cleansing composition. Such compositions comprise a surfactant. The surfactant is preferably included in at least 0.1%, more preferably at least 0.5%, further more preferably at least 1.0% by weight of the composition. The surfactant is preferably included in at most 80%, more preferably at most 75% by weight of the composition. Surfactant for inclusion in the composition of the invention may be more than 2%, 3%, or 5% at the lower end and may be less than 70%, 60% or 50% at the higher end. The composition is a personal cleansing composition, although it may also be used in home care preparations or in food preparations. By a personal care composition is meant that the composition is applied externally i.e. to a topical surface of the body e.g the skin, hair, scalp, oral mucosa, teeth and left thereon for a specified period of time or it may be rinsed off soon after applying the composition on the skin. The various types of personal cleansing compositions in which the preservative system of the invention may be included are described below.

The composition of the invention may be prepared so that it is suitable for use as an oral care or a skin, scalp or hair care product. The product may be delivered in the form of a solid, soft solid, liquid, emulsion, microemulsion, lotion, cream, gel, or aerosol forms. Most preferred forms are an emulsion (like a lotion or a cream) or as a gel. The pH of the composition at the lower end is preferably higher than 3.5, more preferably higher than 4, further more preferably

more than 4.5. At the upper end the pH is preferably lower than 11.0, more preferably lower than 10.0 further more preferably lower than 9.0, even further more preferably less than 8.5. Highly preferred pH range is 4 to 8.5, further more preferred range is from 5 to 7.5.

Oral Care

When the personal cleansing composition of the invention is delivered for oral care, it includes a cosmetically acceptable base which may be an abrasive, a thickener, a humectant or an orally acceptable surfactant. The product may be delivered in the form of an ointment, a gel, a dentifrice or a mouthwash.

Oral care compositions preferably comprise an abrasive. Gels usually contain silica, whereas opaque creams generally contain calcium based abrasives, especially chalk. Preferred toothpaste compositions have 5 to 60 wt% calcium based abrasive. In an preferred embodiment, the composition comprises a thickener. Typically, thickening silica, sodium carboxymethyl cellulose and/or a Carbomer is/are preferred thickeners for use in the composition of the invention. Thickening silica is especially preferred to be used in gel toothpastes. Gel toothpastes generally contain up to 8.5 wt% thickening silica whereas opaque toothpastes typically contain 3 to 4 wt% thickening silica. Thickener, when present, preferably makes up from 0.01 to about 10%, more preferably from 0.1 to 9%, and most preferably, from 1.5 to 8% by weight of the composition.

Suitable humectants are preferably used in the oral care composition of the present invention. Glycerin, polyethylene glycol, sorbitol or mixtures thereof are the preferred humectants. The humectant may be present in the range of from 10 to 90% by weight of oral care compositions. More preferably, the humectant makes up from 25 to 80%, and most preferably, from 45 to 70% by weight of the composition, based on total weight of the composition, Preferably, an oral care composition comprises a surfactant. The preferred anionic surfactants are sodium lauryl sulphate and/or sodium dodecylbenzene sulfonate. Most preferably the surfactant is sodium lauryl sulphate. Water may preferably be included in 5 to 95%, in particular 10 to 75%, and especially at from 10 to 60%, further more preferably 10 to 45% by total weight of the composition.

Hair Care

As per another preferred aspect of the invention, the composition may be used for hair care. One medium through which this may be delivered is that of a shampoo. The composition of the invention especially shampoos are formulated with an anionic surfactant e.g. an alkyl sulphate and/or ethoxylated alkyl sulfate surfactant. These anionic surfactants are preferably present at a level of from 1 to 20%, preferably 2 to 16%. Preferred alkyl sulfates are C8-18 alky sulfates, more preferably C12-18 alkyl sulfates, preferably in the form of a salt with a solubilising cation such as sodium, potassium, ammonium or substituted ammonium. Preferred ethoxylated alkyl sulfate anionic surfactant is sodium lauryl ether sulfate (SLES). SLES having an average degree of ethoxylation of from 0.5 to 3, preferably 1 to 3 is especially preferred. A composition of the invention preferably additionally comprises an amphoteric surfactant preferably a betaine surfactant preferably an alkyl amidopropyl betaine surfactant for example cocamidopropyl betaine. In a preferred embodiment, the composition comprises from 0.1 to 10 wt.%, of a betaine surfactant

To enhance deposition of actives from compositions of the invention especially shampoos, cationic polymers are generally included therein. In the present invention too, it is preferred that the composition additionally includes 0.01 to 2.0% of a cationic polymer. The cationic polymer is preferably guar hydroxypropyl trimonium chloride.

Skin Cleansing

The preservative system of the invention is preferably included in a skin cleansing composition. When the period of time of application is low say of the order of a few seconds to a few minutes after which the composition is rinsed off with water or wiped away, such a composition is known as a skin cleansing composition or a wash-off composition. Such a composition includes any product applied to a human body for also improving appearance, cleansing, odor control or general aesthetics. The composition for skin cleansing of the present invention can be in the form of a liquid, lotion, cream, foam, scrub, gel, bar, handwash, facewash or a bodywash product.

The composition of the invention may be used for skin cleansing e.g. as a hand wash product. The composition comprises a surfactant. A particularly preferred surfactant is soap. Soap is a suitable surfactant for personal washing applications of the antimicrobial composition of the invention. The soap is preferably $C_8$-$C_{24}$ soap, more preferably $C_{10}$-$C_{20}$ soap and most preferably $C_{12}$-$C_{18}$ soap. The cation of the soap can be alkali metal, alkaline earth metal or ammonium. Preferably, the cation of the soap is selected from sodium, potassium or ammonium. More preferably the cation of the soap is sodium or potassium.

A typical fatty acid blend consisted of 5 to 30% coconut fatty acids and 70 to 95% fatty acids by weight of soap. Fatty acids derived from other suitable oils/fats such as groundnut, soybean, tallow, palm, palm kernel, etc. may also

be used in other desired proportions.

When present, the soap, of the present is preferably present in an amount of 1 to 90%, preferably from 10 to 85%, more preferably 25 to 75% by weight of the composition.

Other preferred surfactants are nonionic surfactants, such as C8-C22, preferably C8-C16 fatty alcohol ethoxylates, comprising between 1 and 8 ethylene oxide groups when the product is in the liquid form. The surfactants are preferably selected from primary alkyl sulphate, secondary alkyl sulphonates, alkyl benzene sulphonates, or ethoxylated alkyl sulphates. The composition may further comprise an anionic surfactant, such as alkyl ether sulphate preferably those having between 1 and 3 ethylene oxide groups, either from natural or synthetic source and/or sulphonic acid. Especially preferred are sodium lauryl ether sulphates. Alkyl polyglucoside may also be present in the composition, preferably those having a carbon chain length between C6 and C16.

Preferred compositions may include other known ingredients such as perfumes, pigments, emollients, sunscreens, emulsifiers, gelling agents and thickening agents. Choice of these ingredients will largely depend on the format of the composition.

Water is a preferred carrier. When water is present, it is preferably present in at least 1%, more preferably at least 2%, further more preferably at least 5% by weight of the composition. When water is the carrier, a preferred liquid composition comprises 10 to 99.8% by weight water. The liquid antimicrobial composition is useful as a skin antiseptic liquid, for skin cleansing, in particular for hand wash or a face wash. When water is the carrier, a preferred solid composition comprises 5 to 30% by weight water.

The solid antimicrobial composition is preferably in form of a shaped solid, more preferably a bar. The solid antimicrobial composition is particularly useful for skin cleansing in particular for hand wash or a face wash.

According to another aspect, inorganic particulate material is also a suitable carrier.

When inorganic particulate material is the carrier, the composition is in a solid form. Preferably the inorganic particulate material is talc. When the inorganic particulate material is talc, the solid composition is particularly useful as a talcum powder for application on face or body.

In another aspect of the present invention, the composition of the present invention is suitable for use in wipes for personal hygiene.

The composition of the invention preferably includes 0.01 to 5% of the preservative system by weight of the composition. The composition is preferably a skin cleansing composition.

Personal care compositions may also comprise other ingredients which are common in the art to enhance physical properties and performance. Suitable ingredients include but are not limited to humectants, thickeners, opacifiers, binders, colorants and pigments, pH adjusting agents, optics, perfumes, viscosity modifiers, biological additives, buffering agents, conditioners, natural extracts, essential oils and skin benefit agents including anti-inflammatory agents, cooling agents, antiperspirant agents, anti-aging agents, anti-acne agents, anti-microbial agents and antioxidants.

According to another aspect of the present invention there is provided a method of preserving a composition as defined in the appended set of claims comprising the step of adding to the composition a preservative system of the invention. The two ingredients of the preservative system could be added to the composition sequentially but it is preferred that the two are premixed before addition to the composition to be preserved.

The invention will now be illustrated by means of the following examples

## Examples

The differing behaviours of inhibitory antimicrobials in isolation and mixtures have been widely explored using the concept of the Fractional Concentration and Fractional Inhibitory Concentration (FIC). See for instance JRW Lambert and R Lambert, J. Appl. Microbiol 95, 734 (2003); T. Jadavji, CG Prober and R Cheung, Antimicrobial Agents and Chemotherapy 26, 91 (1984), and WO 2004/006876. These parameters can be defined as follows:

FC (component *a*) = Concentration of component *a* tested in the mixture / MIC (component *a* tested as a single active)

FIC (component *a*) = MIC (component *a* tested in the mixture) / MIC (component *a* tested as a single active)

The interactions between antimicrobials can be additive, synergistic or possibly antagonistic depending on whether the efficacy of the combination is equivalent to, greater than or less than that obtained for the same total concentration of the individual components when tested alone.

These relationships can be expressed mathematically by summing the fractional MIC values for all the components present in the mixture to give the "fractional inhibitory index":

$$\sum FIC = FIC_{\text{(component 1)}} + FIC_{\text{(component 2)}}$$

Such that:

$\Sigma FIC \geq 1$ corresponds to additive or antagonistic activity
$\Sigma FIC < 1$ corresponds to synergistic activity

A comparable method is the calculation of the synergy index (SI) which is an industrial accepted method described by Kull, F.C.; Eisman, P.C.; Sylwestrowicz,H.D. and Mayer, R.L., in Applied Microbiology 9:538-541 (1961).

Liquid broth assays (MIC and checkerboard) were conducted to identify the minimum concentration(s) of individual and binary combinations of preservation chemicals. A modified methodology to ISO 20776-1:2006 was utilised for the screening as follows. Stock solutions of preservation chemicals and tryptic soy broth were inoculated with 1-5$\times 10^6$ microorganisms and incubated at 30°C for 24 hours, after which optical densities at $OD_{600}$nm were measured. MIC was defined as the concentration at which <25% growth was observed in comparison to a positive growth control containing no preservation chemicals. Preservation chemicals were screened at a concentration range of 0.0156-2%.

Table 1: Fractional Inhibitory Concentrations for benzhydroxamic acid with sodium benzoate against two different microbial pools

| Weight Ratio | 1:16 | 1:8 | 1:4 | 1:2 |
|---|---|---|---|---|
| *Pseudomonas aeruginosa, Pseudomonas putida* and Burkholderia cepacia pool | 1.06 | 0.75 | 1.00 | 0.75 |
| *Enterobacter gergoviae* and *Klebsiella species* | - | - | 0.75 | - |

The data in the table above indicates that a combination of benzhydroxamic acid with sodium benzoate provides for synergistic antimicrobial activity against more than one microbial pool. The synergy is especially effective in the weight ratio range benzhydroxamic acid with sodium benzoate of 1:8 to 1:2.

Table 2: Fractional Inhibitory Concentrations for tricarballylic acid with sodium benzoate against two different microbial pools

| Weight Ratio | 1:8 | 1:4 | 1:2 | 1:1 | 2:1 | 4:1 | 5:1 |
|---|---|---|---|---|---|---|---|
| *Pseudomonas aeruginosa, Pseudomonas putida & Burkholderia cepacia* | - | 0.75 | 1.00 | 0.75 | 0.75 | 0.63 | 1.06 |
| *Enterobacter gergoviae & Klebsiella* species | 0.625 | 0.5 | - | - | - | - | - |

The data in the table above indicates that a combination of tricarballylic acid with sodium benzoate provides for synergistic antimicrobial activity against more than one microbial pool. The synergy is especially effective in the weight ratio range of 1:8 to 4:1.

## Claims

**1.** A composition comprising

(a) a preservative system comprising sodium benzoate in combination with a second active selected from benzhydroxamic acid or tricarballylic acid;and
(b) a surfactant; wherein
the weight ratio of the second active to sodium benzoate is in the range of 1:8 to 1:2 when the second active is benzhydroxamic acid; and
wherein the weight ratio of the second active to sodium benzoate is in the range of 1:8 to 4:1 when the second

active is tricarbalyllic acid;
wherein the composition is a personal cleansing composition.

2. A composition as claimed in claim 1 comprising 0.01 to 5% of the preservative system by weight of the composition.

3. A composition as claimed in any one of the preceding claims wherein the pH of the composition is higher than 3.5 and less than 11.0.

4. A method of preserving a composition as claimed in any one of the preceding claims in which the sodium benzoate and the second active are pre-mixed before addition to the composition to be preserved.

## Patentansprüche

1. Zusammensetzung, umfassend

(a) ein Konservierungssystem, umfassend Natriumbenzoat in Kombination mit einem zweiten Wirkstoff, ausgewählt aus Benzhydroxamsäure oder Tricarballylsäure, und
(b) ein Tensid, wobei
das Gewichtsverhältnis des zweiten Wirkstoffs zu Natriumbenzoat in dem Bereich von 1:8 bis 1:2 liegt, wenn der zweite Wirkstoff Benzhydroxamsäure ist, und
wobei das Gewichtsverhältnis des zweiten Wirkstoffs zu Natriumbenzoat in dem Bereich von 1:8 bis 4:1 liegt, wenn der zweite Wirkstoff Tricarballylsäure ist; wobei die Zusammensetzung eine Körperpflegezusammensetzung ist.

2. Zusammensetzung, wie im Anspruch 1 beansprucht, umfassend 0,01 bis 5% des Konservierungssystems, bezogen auf das Gewicht der Zusammensetzung.

3. Zusammensetzung, wie in irgendeinem der vorhergehenden Ansprüche beansprucht, wobei der pH-Bereich der Zusammensetzung mehr als 3,5 und weniger als 11,0 beträgt.

4. Verfahren zum Konservieren einer Zusammensetzung, wie in irgendeinem der vorhergehenden Ansprüche beansprucht, bei dem das Natriumbenzoat und der zweite Wirkstoff vor Zugabe zu der zu konservierenden Zusammensetzung vorgemischt werden.

## Revendications

1. Composition comprenant

(a) un système de conservation comprenant du benzoate de sodium en combinaison avec un second actif choisi parmi l'acide benzhydroxamique ou l'acide tricarballylique ; et
(b) un tensioactif ; dans laquelle
le rapport en masse du second actif au benzoate de sodium se trouve dans l'intervalle de 1:8 à 1:2 lorsque le second actif est l'acide benzhydroxamique ; et
dans laquelle le rapport en masse du second actif au benzoate de sodium se trouve dans l'intervalle de 1:8 à 4:1 lorsque le second actif est l'acide tricarballylique ;
dans laquelle la composition est une composition nettoyante d'hygiène personnelle.

2. Composition selon la revendication 1 comprenant de 0,01 à 5 % du système de conservation en masse de la composition.

3. Composition selon l'une quelconque des revendications précédentes, dans laquelle le pH de la composition est supérieur à 3,5 et inférieur à 11,0.

4. Procédé de conservation d'une composition selon l'une quelconque des revendications précédentes, dans lequel le benzoate de sodium et le second actif sont pré-mélangés avant l'addition à la composition destinée à être conservée.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5250299 A
- US 3446630 A
- US 2018177695 A
- WO 2004006876 A


### Non-patent literature cited in the description


- **JRW LAMBERT ; R LAMBERT.** *J. Appl. Microbiol,* 2003, vol. 95, 734
- **T. JADAVJI ; CG PROBER ; R CHEUNG.** *Antimicrobial Agents and Chemotherapy,* 1984, vol. 26, 91
- **KULL, F.C. ; EISMAN, P.C. ; SYLWESTROWICZ,H.D ; MAYER, R.L.** *Applied Microbiology,* 1961, vol. 9, 538-541